# EUROPEAN PATENT SPECIFICATION

(11) **EP 1 803 466 B1**
(45) Date of publication and mention of the grant of the patent: **11.07.2012**
(21) Application number: 07000627.5
(22) Date of filing: 26.09.2002
(51) Int. Cl.: A61K 39/395, A61P 19/02, A61P 37/06, C07K 16/28, C07K 16/24

(54) **Treatment of chronic joint inflammation by an aglycosylated anti-CD3 antibody**
Behandlung chronischer Gelenksentzündungen von einem aglykosylierten anti-CD3 Antikörper
Traitement pour inflammation articulaire chronique par un anticorps aglycosylé contre CD3

(30) Priority: 26.09.2001 GB 0123156
(43) Date of publication of application: 04.07.2007
(62) Divisional of application: 02765050.6
(73) Proprietor: ISIS INNOVATION LIMITED, Summertown, Oxford OX2 7SG (GB)
(72) Inventor: Isaacs, John, Molecular Medicine Unit, Leeds LS9 7TF (GB); Waldmann, Herman, Oxford OX1 3RE (GB); Hale, Geoffrey, Therapeutic Antibody Centre, Oxford OX3 7JT (GB)
(74) Representative: Brearley, Helen Rebecca

(56) References cited:
- WO-A-00/05268
- WO-A-01/37860
- CA-A- 2 224 256
- US-A- 5 183 657
- C. HUGHES ET AL.: "Suppression of collagen-induced arthritis in mice with anti-CD3 F(ab')2 fragments." THE JOURNAL OF IMMUNOLOGY, vol. 150, no. 8(2), 21 May 1993 (1993-05-21), page 81A, XP008079212 Baltimore, MD, U.S.A.
- A. MALFAIT ET AL.: "Chronic relapsing homologous collagen-induced arthritis in DBA/1 mice as a model for tresting disease-modifying and remission-inducing therapies." ARTHRITIS AND RHEUMATISM, vol. 44, no. 5, May 2001 (2001-05), pages 1215-1224, XP002254664 New York, NY, USA
- KINNE R. ET AL: 'Review: Macrophages in rheumatoid arthritis.' ARTHRITIS RESEARCH vol. 2, no. 3, 2000, pages 189 - 202
- FIRESTEIN G.; ZVAIFLER N.: 'How important are T cells in chronic rheumatoid synovitis? II. T cell-independent mechanisms from beginning to end.' ARTHRITIS & RHEUMATISM vol. 46, no. 2, February 2002, pages 298 - 308

## Description

### Field of the Invention

The present invention relates to the treatment of chronic joint inflammation, particularly chronic inflammatory synovitis, more particularly rheumatoid arthritis and related conditions, using antibodies targeted at the CD3 antigen.

### Background to the Invention

Rheumatoid arthritis is a chronic inflammatory disease affecting the joints and other tissues and has an annual incidence of from 1 to 10 per 10,000 of the adult population in developed countries and a prevalence of approximately 1%. Apart from the pain and suffering brought about by the condition, the progressive nature of the disease has a significant effect on morbidity and mortality with a standard mortality ratio of up to 3.0. The annual cost of the disease in the USA alone has been estimated at well over $1 billion (Brooks, The Lancet, 30th January 1993, page 286). Present methods of treatment of the disease are far from satisfactory and problems include unpredictability of response and the fact that many drugs are not well tolerated and/or have potentially dangerous adverse effects. There are a number of other related "rheumatic" diseases with comparable inflammation of synovial tissue, including ankylosing spondylitis, psoriatic arthritis and some forms of osteoarthritis that can also be considered under the heading of chronic synovial inflammation.

WO 93/97899 relates to the treatment of T-cell mediated inflammation of the joints using an antibody recognizing the CD_{w}52 antigen, alone or in combination with an anti-CD4 antibody. Particularly described is the use of a humanised monoclonal antibody, Campath-1H, to treat rheumatoid arthritis. While such therapy offers the hope of significant improvement, it involves severe depletion of systemic T lymphocytes. While patients' levels of T-lymphocytes are low, and thus in theory at least they are immuno-compromised and vulnerable to infection, symptoms of the disease return. The balance between risk of infection and therapeutic effectiveness makes the use of Campath-1H unattractive to physicians for chronic conditions such as rheumatoid arthritis. Anti-CD4 antibodies have also been suggested by others for treatment of chronic joint inflammation but thus far such antibodies have not been demonstrated as adequately effective in the clinic.

Discussion of the CD3 antigen can be found for example in the report of the First International Workshop and Conference on Human Leukocyte Differentiation Antigens. Various glycosylated antibodies directed against the CD3 antigen are also described in the reports of this series of Workshops and Conferences, particularly the Third and Fourth, published by Oxford University Press: Anti-CD3 antibodies have thus been known for around 20 years, but have had only a limited use as immunosuppressive agents in, for example, the treatment of rejection episodes following the transplantation of renal, hepatic and cardiac allografts, due to first dose reactions which can be severe.

It is known that anti-CD3 monoclonal antibodies can be used to sensitise T-cells to secondary proliferative stimuli such as IL1 (interleukin 1) and IL2 (interleukin 2). In addition, certain CD3 monoclonal antibodies are themselves mitogenic for T-cells. This property is isotype dependent and results from the interaction of the CD3 antibody Fc domain with Fc receptors on the surface of accessory cells. Rodent CD3 antibodies have been used to influence immunological status by suppressing, enhancing or re-directing T-cell responses to antigens.

US 5,968,509 relates to humanized monoclonal antibodies targeted at CD3 and describes their use to control graft rejection and treat lymphomas. US 5,585,097 relates to improved anti-CD3 antibodies of the type described in US 5,968,509, in so far as they are aglycosylated and thus have reduced side effects associated with 'first dose response' when used in therapy.

Malfait et al, Arthritis and Rheumatism Vol 44, No 5, May 2001, pp 1215-1224 relates to chronic relapsing collagen-induced arthritis in DBA/1 mice as a model for testing disease modifying and remission inducing therapies. One therapy investigated was combined therapy of anti-CD3 antibody plus anti-tumor necrosis factor antibody administered at the onset of symptoms which blocked the progression of arthritis in the mouse for the rest of the study period of 56 days.

Hughes et al, J. Immunol., 150, no. 8(2), page 81A, Abstract 450 also relates to collagen-induced arthritis in DBA/1 mice as a model for rheumatoid arthritis. Simultaneous administration of anti-CD3 F(ab')2 fragments with the collagen resulted in a significant decrease in disease severity.

CA-2 224 256-A1 relates to the treatment of auto-immune disease in a mammal with a non-nitrogenic anti-CD3 active principle. Treatment of overtly diabetic NOD mice with purified F(ab')2 fragments of anti-CD3 monoclonal antibody is disclosed. The effect of the fragments on cytokine gene transcription and cytokine production by stimulated spleen cells in the mice is also reported.

US-5 183 657 relates to the use of antibodies against human TNFα to prevent or treat shock-related conditions arising from anti-lymphocyte antibody therapy for example with OKT3.

The murine model described above, is very much dependent on an antigen-driven stimulus to provoke T-cell reactivity. No such eliciting antigens have yet been identified for human rheumatoid arthritis, ankylosing spondylitis or psoriatic arthropathy. In the absence of indicators that the murine disease is comparable to the human synovial disorders, it is not possible therefore, to extrapolate from data on therapeutic benefits to human rheumatoid arthritis and the related synovial inflammatory disorders.

### Summary of the Invention

The present invention provides an aglycosylated anti-CD3 antibody for use in the treatment of chronic joint inflammation.

As used herein the following terms have the definitions stated:
"CD3" means Cluster Designation 3;
"aglycosylated" means here an antibody which has lost its major glycosylation site by a mutation of asparagine at position 297 to alanine;
"first dose reaction" means a cytokine release reaction which is known to follow the first dose administration of CD3 antibodies, and often associated with significant morbidity and discomfort for the patient; "affinity" in relation to an antibody means the ability of each of the binding arms of the antibody to bind to their ligand (the antigen).

The treatment according to the invention using an aglycosylated anti-CD3 antibody has the major advantage that only a short-term treatment is required to provide sustained long-term benefits. For example treatment may be carried out for a period of up to 2 weeks, for example 1 to 5 days, without subsequent re-administration for at least 6 months. This treatment provides sustained long term benefit to the patient over a period of at least 6 months, and in many cases much longer, for example up to 24 months or more.

The use of an aglycosylated anti-CD3 antibody according to the present invention also has the advantage that it does not result in the depletion of T-lymphocytes, as is the case with Campath 1-H.

Therapy with an aglycosylated anti-CD3 antibody according to the invention is such that the first dose reactions associated with anti-CD3 antibodies are avoided or reduced at least to a level which can be tolerated by the patient. In accordance with the invention, first dose reactions are reduced by modification of the antibody, specifically by aglycosylation. Such mutation in the Fc region of the antibody is designed to prevent binding to Fc receptors.

Optionally, the antibody may be administered with another substance which reduces the reaction. Examples of substances which can reduce first dose reactions when administered with an anti-CD3 antibody are prophylaxis with steroids and antihistamines.

The anti-CD3 antibody can be administered with other therapeutic agents for treating chronic joint inflammation and the antibody can be used together with such agents as part of an overall therapy for the condition. Examples of such other therapeutic agents include steroids, anti-TNF antibodies. Many therapies for chronic joint inflammation are immunosuppressive and these may also be effective in reducing first dose reaction to the anti-CD3 antibody. There may also be a time lag between administration of the anti-CD3 antibody and the patient experiencing any therapeutic benefit and this time lag may run into weeks. Use of the anti-CD3 antibody as part of an overall strategy together with other therapeutic agents may assist in alleviating symptoms of the condition during this time lag.

### Detailed description of the Invention

WO-00/05268 discloses the full sequence of the preferred chimeric anti CD3 antibody, which is part humanized, suitable for use in the present invention.

Preferred antibodies for use in the present method are one or more of humanized monoclonal antibodies, aglycosylated antibodies and antibodies having the CDRs contained in the antibodies OKT3 and YTH 12.5.14.2.
The antibody OKT3 is discussed in publications such as Chatenoud et al., Transplantation, 1991, 51, 334 and the New England Journal of Medicine paper, 1985, 313, 339, and also in European Patent No. 0 018 795 and US Patent No. 4,361,539. The antibody YTH 12.5.14.2 (hereinafter referred to as YTH 12.5) is discussed in publications such as Cobbold, S.P. & Waldmann, H. 1984 Nature 308, 460-462. and Clark et al., European J. Immunol., 1989, 19, 381-388 and reshaped YTH 12.5 antibodies are the subject of US 5,968,509 and its equivalents, this application describing in detail the CDRs present in this antibody. While the present invention is not limited to use of any particular anti-CD3 antibody, the antibodies of the invention preferably have at least one CDR selected from the amino acid sequences:
(a) Ser-Phe-Pro-Met-Ala (SEQUENCE ID NO. 1),
(b)
(c) Phe-Arg-Gln-Tyr-Ser-Gly-Gly-Phe-Asp-Tyr (SEQUENCE ID NO. 3).
(d) Thr-Leu-Ser-Ser-Gly-Asn-Ile-Glu-Asn-Asn-Tyr-Val-His (SEQUENCE ID NO.4).
(e) Asp-Asp-Asp-Lys-Arg-Pro-Asp (SEQUENCE ID NO. 5),
(f) His-Ser-Tyr-Val-Ser-Ser-Phe-Asn-Val (SEQUENCE ID NO. 6),
and conservatively modified variants thereof

The term "conservatively modified variants" is one well known in the art and indicates variants containing changes which are substantially without effect on antibody-antigen affinity.

The CDRs are situated within framework regions of the heavy chain for (a), (b) and (c)) and light chain for (d), (e) and (f) variable domains. The antibody also comprises a constant domain.

In a preferred embodiment the aglycosylated antibody has three CDRs corresponding to the amino acid sequences (a), (b) and (c) above or conservatively modified variants thereof and/or three CDRs corresponding to amino acid sequences 5 (d), (e) and (f) or conservatively modified variants thereof the heavy chain CDRs (a), (b) and (c) being of most importance.

A preferred aglycosylated antibody for the present use or method having a binding affinity for the CD3 antigen thus has a heavy chain with at least one CDR and particularly three CDRs selected amino acid SEQUENCE ID NOS 1 to 6 (a to f) and conservatively modified variants thereof

Where an aglycosylated antibody according to the invention contains preferred CDRs as described hereinbefore it conveniently contains both one or more of the specified heavy chain CDRs and one or more of the specified light chain CDRs. The CDRs (a), (b) and (c) are arranged in the heavy chain in the sequence: framework region 1/(a)/framework region 2/(b)/framework region 3/(c)/framework region 4 in a leader → constant domain (N-terminal to C-terminal) direction and the CDRs (d), (e) and (f) are arranged in the light chain in the sequence: framework region 1/(d)/framework region 2/(e)/framework region 3/(f)/framework region 4 in a leader → constant domain direction. It is preferred, therefore, that where all three are present the heavy chain CDRs are arranged in the sequence (a), (b), (c) in a leader → constant domain direction and the light chain CDRs are arranged in the sequence (d). (e). (f) in a leader → constant domain direction.

It should be appreciated however, that aglycosylated antibodies for the use according to the invention may contain quite different CDRs from those described hereinbefore and that, even when this is not the case, it may be possible to have heavy chains and particularly light chains containing only one or two of the CDRs (a), (b) and (c) and (d), (e) and (f) respectively. However, although the presence of all six CDRs defined above is therefore not necessarily required in an aglycosylated antibody according to the present invention, all six CDRs will most usually be present in the most preferred antibodies.

A particularly preferred aglycosylated antibody therefore has a heavy chain with three CDRs comprising the amino acid sequences (a), (b) and (c) or conservatively modified variants thereof and a light chain with three CDRs comprising the amino acid sequences (d), (e) and (f) or conservatively modified variants thereof in which the heavy chain CDRs are arranged in the order (a), (b), (c) in the leader constant region direction and the light chain CDRs are arranged in the order (d), (e), (f) in the leader constant region direction.

The CDRs may be of different origin to the variable framework region and/or to the constant region and, since the CDRs will usually be of rat or mouse origin, this is advantageous to avoid an antiglobulin response in the human, although the invention does extend to antibodies with such regions of rat or mouse origin.

More usually the CDRs are either of the same origin as the variable framework region but of a different origin from the constant region, for example in a part human chimaeric antibody, or, more commonly, the CDRs are of different origin from the variable framework region.

The preferred CDRs discussed hereinbefore are obtained from a rat CD3 antibody. Accordingly, although the variable domain framework region can take various forms, it is conveniently of or derived from those of a rodent, for example a rat or mouse, and more preferably of or derived from those of human origin. One possibility is for the antibody to have a variable domain framework region corresponding to that in the YTH I2.5 hybridoma although the constant region will still preferably be of or derived from those of human origin. However the antibody of the invention is preferably in the humanised form as regards both the variable domain framework region and as discussed further hereinafter, the constant region, or other non-immunogenic forms

Accordingly, the invention further comprises use of an aglycosylated antibody which has a binding affinity for the human CD3 antigen and in which the variable domain framework regions and/or the constant region are of or are derived from those of human origin. Certain human variable domain framework sequences will be preferable for the grafting of the preferred CDR sequences, since the 3-dimensional conformation of the CDRs will be better maintained in such sequences and the antibody will retain a high level of binding affinity for the antigen. Desirable characteristics in such variable domain frameworks are the presence of key amino acids which maintain the structure of the CDR loops in order to ensure the affinity and specificity of the antibody for the CD3 antigen, the lambda type being preferred for the light chain.

Human variable region frameworks which are particularly suitable for use in conjunction with the above CDRs have been previously identified in US 5,968,509. The heavy chain variable (V) region frameworks are those coded for by the human VH type III gene VH26.D.J. which is from the B cell hybridoma cell line 18/2 (Genbank Code: Huminghat. Dersimonian et al., Journal of Immunologv. 139, 2496 2501). The light chain variable region frameworks are those of the human VLλ type VI gene SUT (Swissprot code; LV6CSHum, Solomon et al. In Glenner er al (Eds), Amyloidos's, Plenum Press N.Y., 1986, p.449.

The one or more preferred CDRs of the heavy chain of the rat anti-CD3 antibody are therefore preferably present in a human variable domain framework which has the following amino acid sequence reading in the leader ->(arrow) constant region direction, CDR. indicating a CDR (a), (b) or (c) as defined hereinbefore, a conservatively modified variant thereof or an alternative CDR:-

In an aglycosylated antibody containing all three preferred CDRs, the heavy chain variable region comprises the following sequence:-

Similarly, the one or more preferred CDRs of the light chain of the rat CD3 antibody are therefore preferably present in a human variable domain framework which has the following amino acid sequence reading in the leader → constant region direction, CDR indicating a CDR (d), (e) and (t) as defined hereinbefore, a conservatively modified variant thereof or an alternative CDR:-

In an aglycosylated antibody containing all three preferred CDRs the light chain variable region comprises the following sequence:-

The variable domains, for example comprising one or more preferred CDRs as described above, preferably in the humanised form having human antibody-derived variable framework regions, are attached to appropriate constant domains.

The heavy and light chain constant regions can be based on antibodies of different types as desired subject to the antibody being an IgG antibody, but although they may be of or derived from those of rat or mouse origin they are preferably of or are derived from those of human origin. For the light chain the constant region is preferably of the lambda type and for the heavy chain it is preferably of an IgG isotype, especially IgGl, modified to effect aglycosylation as appropriate. All human constant regions of the IgG isotype are known to be glycosylated at the asparagine residue at position 297, which makes up part of the N-glycosylation motif Asparagine297- X298 - Serine299 or Threonine299, where X is the residue of any amino acid except proline. The antibody of the invention may thus be aglycosylated by the replacement of Asparagine297 in such a constant region with another amino acid which cannot be glycosylated. Any other amino acid residue can potentially be used, but alanine is the most preferred. Alternatively, glycosylation at Asparagine297 can be prevented by altering one of the other residues of the motif, e.g. by replacing residue 298 by proline, or residue 299 by any amino acid other than serine or threonine. Techniques for performing this site directed mutagenesis are well known to those skilled in the art and may for example be performed using a site directed mutagenesis kit such, for example, as that commercially available from Amersham. The procedure is further exemplified hereinafter.

It is well recognized in the art that the replacement of one amino acid in a CDR with another amino acid having similar properties, for example the replacement of a glutamic acid residue with an aspartic acid residue, may not substantially alter the properties or structure of the peptide or protein in which the substitution or substitutions were made. Thus, the aglycosylated antibodies for use in the present invention include those antibodies containing the preferred CDRs but with a specified amino acid sequence in which such a substitution or substitutions have occurred without substantially altering the binding affinity and specificity of the CDRs. Alternatively, deletions may be made in the amino acid residue sequence of the CDRs or the sequences may be extended at one or both of the N- and C-termini whilst still retaining activity.

Preferred aglycosylated antibodies according to the present invention are such that the affinity constant for the antigen is 10⁵ mole⁻¹ or more, for example up to 10¹² mole⁻¹. Ligands of different affinities may be suitable for different uses so that, for example, an affinity of 10⁶, 10⁷ or 10⁸ mole⁻¹ or more may be appropriate in some cases. However antibodies with an affinity in the range of 10⁶ to 10⁸ mole⁻¹ will often be suitable. Conveniently the antibodies also do not exhibit any substantial binding affinity for other antigens. Binding affinities of the antibody and antibody specificity may be tested by assay procedures such as those described in the Examples section hereinafter, (Effector Cell Retargetting Assay), or by techniques such as ELISA and other immunoassays.

Antibodies according to the invention are aglycosylated IgG CD3 antibodies having a "Y" shaped configuration which may have two identical light and two identical heavy chains and are thus bivalent with each antigen binding site having an affinity for the CD3 antigen. Alternatively, the invention is also applicable to antibodies in which only one of the arms of the antibody has a binding affinity for the CD3 antigen. Such antibodies may take various forms. Thus the other arm of the antibody may have a binding affinity for an antigen other than CD3 so that the antibody is a bispecific antibody, for example as described in U.S. Patent No. 4,474,893 and European Patent Applications Nos. 87907123.1 and 87907124.9. Alternatively, the antibody may have only one arm which exhibits a binding affinity, such an antibody being termed "monovalent".

Monovalent antibodies (or antibody fragments) may be prepared in a number of ways. Glennie and Stevenson (Nature, 295, 712-713, (1982)) describe a method of preparing monovalent antibodies by enzymic digestion. Stevenson et al. describe a second approach to monovalent antibody preparation in which enzymatically produced Fab' and Fe fragments are chemically cross-linked (Anticancer Drug Design, 3, 219-230 (1989)). In these methods the resulting monovalent antibodies have lost one of their Fab' arms. A third method of preparing monovalent antibodies is described in European Patent No. 131424. In this approach the 'Y' shape of the antibody is maintained, but only one of the two Fab' domains will bind to the antigen. This is achieved by introducing into the hybridoma a gene coding for an irrelevant light chain which will combine with the heavy chain of the antibody to produce a mixture of products in which the monovalent antibody is the one of interest.

More preferably, however, the monovalent aglycosylated CD3 antibodies for the use of the invention are prepared by the following method. This involves the introduction into a suitable expression system, for example a cell system as described hereinafter, together with genes coding for the heavy and light chains, of a gene coding for a truncated heavy chain in which the variable region domain and first constant region domain of the heavy chain are absent, the gene lacking the exon for each of these domains. This results in the production by the cell system of a mixture of (a) antibodies which are complete bivalent antibodies. (b) antibody fragments consisting only of two truncated heavy chains (i.e. an Fc fragment) and (c) fragments of antibody which are monovalent for the CD3 antigen, consisting of a truncated heavy chain and a light chain in association with the normal heavy chain. Such an antibody fragment (c) is monovalent since it has any only one Fab' arm. Production of a monovalent antibody in the form of such a fragment by this method is preferred for a number of reasons. Thus, the resulting antibody fragment is easy to purify from a mixture of antibodies produced by the cell system since, for example, it may be separable simply on the basis of its molecular weight. This is not possible in the method of European Patent No. 131424 where the monovalent antibody produced has similar characteristics to a bivalent antibody in its size and outward appearance.

Additionally, the production of a monovalent antibody fragment by the new method uses conditions which can more easily be controlled and is thus not as haphazard as an enzyme digestion/chemical coupling procedure which requires the separation of a complex reaction product, with the additional advantage that the cell line used will continue to produce monovalent antibody fragments, without the need for continuous synthesis procedures as required in the enzyme digestion/chemical coupling procedure.

Aglycosylated antibodies for the use or method of may in general be produced synthetically in a number of ways. Most conveniently, however, appropriate gene constructs for the constant and variable regions of the heavy and light chains which are present in the antibody are separately obtained and then inserted in a suitable expression system.

Genes encoding the variable domains of a ligand of the desired structure may be produced and conveniently attached to genes encoding the constant domains of an antibody which have undergone site directed mutagenesis. These constant genes may be obtained from hybridoma cDNA or from the chromosomal DNA and have undergone mutagenesis (site directed) to produce the aglycosylated constant regions. Genes encoding the variable regions may also be derived by gene synthesis techniques used in the identification of the CDRs contained herein. Suitable cloning vehicles for the DNA may be of various types.

Expression of these genes through culture of a cell system to produce a functional CD3 ligand is most conveniently effected by transforming a suitable prokaryotic or particularly eukaryotic cell system, particularly an immortalized mammalian cell line such as a myeloma cell line, for example the YB2/3.01/Ag2O (hereinafter referred to as YO) rat myeloma cell, or Chinese hamster ovary cells (although the use of plant cells is also of interest), with expression vectors which include DNA coding for the various antibody regions, and then culturing the transformed cell system to produce the desired antibody- Such general techniques of use for the manufacture of ligands according to the present invention are well known in the very considerable art of genetic engineering and are described in publications such as "Molecular Cloning" by Sambrook, Eritsch and Maniatis, Cold Spring Harbour Laboratory Press, 1989 (2nd edition). The techniques are further illustrated by the Examples contained herein.

Antibodies for use in accordance with the invention may be formulated for administration to patients by administering the said antibody together with a physiologically acceptable diluent or carrier. The antibodies are preferably administered in an injectable form together with such a diluent or carrier which is sterile and pyrogen free. The dose of antibody to be administered to a patient will depend on the condition of the patient and will be at the discretion of the attendant physician. For example, individual doses of about 1 to 40mg daily, preferably 10 to 30mg daily, may be used. The total dose over a period of, for example, up to 10 days may be in the range of about 50 to 100mg, preferably about 60 to 80mg.

The therapeutic benefit of the therapy according to the invention can be assessed by means of reduction in local/systemic inflammation with a concomitant reduction in pain and improvement in functional capacity and quality of life. This can be assessed by EULAR or ACR response criteria (see Van Gestel et al, Arthritis Rheum., 1996; 39: 34-40 and Felson et al, Arthritis Rheum., 1995; 38: 727-35).
The invention is illustrated by the following examples.

### Example 1 - Production of antibody

A chimeric form of the aglycosyl CD3 antibody was produced using PCR assembly to link the rat CD3 light chain variable region to the human lambda constant region using primers which introduce restriction enzyme sites Hind III and EcoR1 to allow cloning into the Celltech expression vector PEE12 (see Bebbington et al (1992) Biotechnology 10, 169). The primer sequences to allow chimeric form of CD3 light chain to be cloned into PEE12 are as follows: EcoR1 primer GAT GCT GAA TTC TGC AGC TCT AGT CTC CCG TGG TGG [SEQUENCE ID NO 18]
The final construct was sequenced and cloned into PEEI2 already containing the humanised CD3 aglycosyl heavy chain and this was transfected into the myeloma cell line N50 (ECACC No 851 10503-Galfre and Milstein (1981) Enzymology 73 (B) 3-46) by electroporation. Resultant clones were screened for antibody production using ELISA for human IgG 1 and human lambda light chain and on the FACS for binding to human I-cell clone Jurkat cell line. The ELISA uses goat anti-human IgFc (Sigma 12136) as capture antibody and Biotinylated sheep anti-human IgG (Amersham RPN 1003) or Biotinylated goat anti-human lambda light chain (Amersham RPN 1188) as detector antibody (see Routledge et al Eur. 1. Immunol (1991) 21: 2717-2725).

After one transfection 16 clones expressed 60µg/ml to 100µg/ml, transfectants were then cloned by limiting dilution cloning and some of these improved to 120µg/ml. These remained stable in long term culture and large scale antibody production with no problems with cell growth.

### Example 2

An aglycosylated divalent anti-CD3 antibody having humanised heavy chain, rat variable light chain containing CDRs corresponding in sequence to those from the YTH 12.5 rat antibody, and a humanised lambda constant region as described in Example 1 of PCT/GB99102380 (see Example 1 above) was administered in aqueous carrier, ie. normal saline, at a dose of 70mg, over 5 days to four patients.

### Patient 1

The patient was a 50 year old female with a 5 year history of sero-positive rheumatoid arthritis. There were no extra articular manifestations but she also had Felty's syndrome. Previous therapies included sulphasalazine and cyclosporin A. On recruitment to the study she was taking methotrexate 20mg a week and prednisolone 20mg per day. She was severely disabled by her disease and had lost her job as a dental nurse. She was unable to dress herself or wash her hair. She also found it difficult to stand up from a chair or get in and out of bed. She was unable to cut her own meat or open a carton of milk. Bathing was difficult as was reaching and bending. She was unable to grip things such as taps or jars and unable to shop for herself.

Aglycosyl anti-CD3 was administered over 5 days. 30mg was prescribed on day 1 and then 10mg on the subsequent 4 days. No pre-medication was given. The first infusion was complicated by nausea and vomiting, fever reached 40.5°C and was associated with hypotension and diarrhoea. A plasma expander was administered. These symptoms lasted for 36 hours and the second infusion was delayed for 24 hours. By the second infusion however there was already a reduction in pain and inflammation of affected joints. The subsequent 4 infusions were given without complication

Her rheumatoid arthritis flared on day 43 and was treated with a single dose of intra-muscular depomedrone. Her baseline anti rheumatoid medication was continued during anti-CD3 treatment. It is now 21 months since this patient received therapy. She has required no additional medication for her rheumatoid arthritis and actually feels much better than at baseline. Her disease is not in remission but she is now able to live a relatively normal existence and perform many tasks for herself of which she was previously incapable. The number of painful and tender joints is markedly reduced, as is her overall score. Prior to therapy her CRP was consistently between 100 and 200mg/L. It now ranges from 40 to60mg/L. Her lymphocyte count is within the normal range.

### Patient 2

The patient was a 62 year old male with a 30 year history of sere-positive nodular rheumatoid arthritis. Previous therapies included intra-muscular gold, penicillamine, and methotrexate. He had never received oral steroids. At the time of entry to the study he was taking methotrexate 25mg intra-muscularly once weekly and cyclosporin A 150mg daily. He was less disabled than patient 1 but described some difficulty with the majority of daily tasks. He was prescribed a treatment regime of 10mg of antibody on day 1 and then 20mg on the subsequent 4 days. The plan was to infuse the first dose slowly. He was advised to stop cyclosporin A but to continue methotrexate during therapy.

On day 1 a rigor developed after 1 hour and at 8 hours he had developed diarrhoea and vomiting which lasted for a further 12 hours and was associated with a minor fall in blood pressure. These symptoms were treated with hydrocortisone and pethidine. On day 2 he developed a wide spread macular rash and this lasted for several weeks. The full treatment course was administered over the intended time course. There were no acute adverse events on days 2-5.

Unlike patient 1 there was no acute benefit of the treatment and on day 22 his disease worsened, requiring an intra-muscular dose of methylprednisolone. His joint pains were refractory to treatment with parenteral steroids. On day 42 he was commenced on sulphasalazine, 1g bd, and prednisolone 7.5mg daily. Intra-muscular methotrexate was continued. Sulphasalazine therapy was stopped on approximately day 150 as a result of headaches and he received a single intra-muscular injection of 80mg of triamcinolone. From this point onwards his disease improved substantially. He described a complete abatement of his symptoms, and an ability to lead a normal life with no tender or swollen joints. His CRP prior to treatment varied between 70-90mg/L. 18 months from treatment it is usually within the normal range (<10 mg/L) or marginally elevated. The rheumatoid factor titre has also fallen from 1:1280 to 1:40. Compared with prior to anti-CD3, he is now also taking prednisolone 7.5mg per day but is currently tapering the dose of this medication. Furthermore, prednisolone was not highly effective when commenced on day 42.

### Patient 3

The patient was an elderly gentlemen refractory to all standard rheumatoid therapies who also had ischaemic heart disease, and chronic obstructive airways disease. He was severely disabled. The plan was as per patient two but pre-med on day one with methylprednisolone.

Day one with methylprednisolone pre-med produced no reaction. On day 2 with no pre-med the patient developed severe diarrhoea and shortness of breath. Infusion was stopped after approximately a quarter had been infused, and no further infusions were given. There is no short or long-term evidence of efficacy (now at day approx 300).

### Patient 4

The patient was a 58 year old female with sero-positive rheumatoid arthritis with a 30 year duration. She had previously tried and failed penicillamine, sulphasalazine, cyclosporin A, hydroxychloroquine, methotrexate, leflunomide. In addition to rheumatoid arthritis she also suffered with diet controlled diabetes and possible angina. The only therapy she was taking for her rheumatoid arthritis on admission was prednisolone 2.5mg daily. She was significantly disabled by her disease and required help with dressing and was also limited in her mobility. She rarely left the house. She could climb stairs and struggled with washing and dressing. Her therapy was planned as 10mg of antibody on days 1-3 and 20mg on days 4-5. The first dose was preceded by 50mg of a soluble TNF receptor human IgG 1 fusion protein as prophylaxis against a first dose reaction. During the first infusion she developed bronchospasm with some chest pain. She became very anxious and peripherally cyanosed. Blood pressure reached 225/100 mmHg. This reaction was treated with nebulized salbutamol and intravenous hydrocortisone. She subsequently developed rigors. Her temperature reached 39°C during the infusion. Her second infusion was pre-medicated using methylprednisolone and was uncomplicated, as was the third infusion. On day 4 she awoke with breathlessness and chest tightness and subsequently developed mild cardiac failure with an ischaemic ECG. Her subsequent echo-cardiogram showed moderate mitral regurgitation with a dilated left atrium. There was also mild aortic regurgitation into a slightly dilated left ventricle. The cardiac enzyme profile was normal during the days of the infusions and so there was no evidence of myocardial infarction at the time of anti-CD3. Despite clinical improvement in her RA activity (possibly secondary to the TNFR-Ig pre-medication), she did not receive infusions 4 and 5.

At about 2 weeks she developed severe joint pains and her subsequent course was quite similar to patient 2 in the short term. Her joint symptoms proved refractory to parenteral steroids and ultimately she was commenced on prednisolone 20mg daily on day 35. She was most recently seen on day 90. She is currently taking prednisolone 10mg per day. Her CRP ran between 100-200 pre treatment, immediately after treatment it fell to 30mg per litre on day 7 (which may reflect the concurrent TNF-alpha blockade). The CRP has subsequently climbed back to levels close to baseline. She developed a lymphocytosis which was first evident around day 23 but which subsequently resolved.

Four patients were thus treated with aglycosyl anti-CD3. Two of these completed therapy (70 mg antibody in total) and appear to have made a good long-term response. Patient 3 received less than 15mg divided between two infusions and there have been no obvious beneficial effects. Patient 4 received 25-30 mg over three doses. At three months there is no evidence of therapeutic efficacy but follow up is continuing.

## Claims

1. An aglycosylated anti-CD3 antibody for use in the treatment of chronic joint inflammation.

2. An anti-CD3 antibody for use in the treatment according to claim 1, wherein the treatment involves administration of the antibody over a period of up to 2 weeks without subsequent re-administration after this period for at least 6 months.

3. An anti-CD3 antibody for use in the treatment according to claim 2, wherein the treatment involves administration of the antibody over a period of 1 to 5 days.

4. An anti-CD3 antibody for use in the treatment according to any of the preceding claims for the treatment of chronic inflammatory synovitis.

5. An anti-CD3 antibody for use in the treatment according to any of the preceding claims for the treatment of rheumatoid arthritis.

6. An anti-CD3 antibody for use in the treatment according to any of the preceding claims wherein the treatment results in reduced local and systemic inflammation.

7. An anti-CD3 antibody for use in the treatment according to claim 6, wherein the treatment is **characterised in that** local and systemic inflammation is substantially reversed.

8. An anti-CD3 antibody for use in the treatment according to any of the preceding claims, wherein the treatment involves administration of the antibody at a total dosage of 50 to 100mg.

9. An anti-CD3 antibody for use in the treatment according to any of the preceding claims, wherein the treatment involves administration of the antibody in conjunction with a further therapeutic agent for the treatment of chronic joint inflammation.

10. An anti-CD3 antibody for use in the treatment according to claim 9, wherein the treatment involves administration of the antibody with a steroid or an anti-TNF antibody.

11. An anti-CD3 antibody for use in the treatment according to any one of claims 1 to 10 wherein the antibody is in the form of a pharmaceutical composition also comprising a physiologically acceptable diluent or carrier.

## Patentansprüche

1. Aglykosylierter Anti-CD3 Antikörper zur Verwendung in der Behandlung chronischer Gelenkentzündung

2. Anti-CD3 Antikörper zur Verwendung in der Behandlung gemäß Anspruch 1 worin die Behandlung die Verabreichung des Antikörpers über einen Zeitraum von bis zu 2 Wochen ohne nachfolgende Wiederverabreichung nach diesem Zeitraum für mindestens 6 Monate umfasst,

3. Anti-CD3 Antikörper zur Verwendung in der Behandlung gemäß Anspruch 2, worin die Behandlung die Verabreichung des Antikörpers über einen Zeitraum von 1 bis 5 Tagen umfasst.

4. Anti-CD3 Antikörper zur Verwendung in der Behandlung gemäß einem der vorhergehenden Ansprüche zur Behandlung von chronisch entzündlicher Synovitis.

5. Anti-CD3 Antikörper zur Verwendung in der Behandlung gemäß einem der vorhergehenden Ansprüche zur Behandlung von rheumatischer Arthritis.

6. Anti-CD3 Antikörper zur Verwendung in der Behandlung gemäß einem der vorhergehenden Ansprüche, worin die Behandlung zu reduzierter lokaler und systemischer Entzündung führt.

7. Anti-CD3 Antikörper zur Verwendung in der Behandlung gemäß Anspruch 6, worin die Behandlung **dadurch** charakterisiert ist, dass die lokale und systemische Entzündung im Wesentlichen rückgängig gemacht ist.

8. Anti-CD3 Antikörper zur Verwendung in der Behandlung gemäß einem der vorhergehenden Ansprüche, worin die Behandlung die Verabreichung des Antikörpers mit einer Gesamtdosis von 50 bis 100 mg umfasst.

9. Anti-CD3 Antikörper zur Verwendung in der Behandlung gemäß einem der vorhergehenden Ansprüche, worin die Behandlung die Verabreichung des Antikörpers in Verbindung mit einem weiteren therapeutischen Mittel zur Behandlung von chronischer Gelenkentzündung umfasst.

10. Anti-CD3 Antikörper zur Verwendung in der Behandlung gemäß Anspruch 9, worin die Behandlung die Verabreichung des Antikörpers mit einem Steroid oder einem Anti-TNF Antikörper umfasst.

11. Anti-CD3 Antikörper zur Verwendung in der Behandlung gemäß einem der Ansprüche 1 bis 10, worin der Antikörper in Form einer pharmazeutischen Zusammensetzung vorliegt, die außerdem ein physiologisch annehmbares Verdünnungsmittel oder Trägermaterial umfasst.

## Revendications

1. Anticorps anti-CD3 aglycosylé à utiliser dans le traitement d'une inflammation articulaire chronique.

2. Anticorps anti-CD3 à utiliser dans le traitement selon la revendication 1, dans lequel le traitement implique l'administration de l'anticorps pendant un laps de temps s'étendant jusqu'à 2 semaines en l'absence d'une réadministration ultérieure après cette période pendant au moins 6 mois.

3. Anticorps anti-CD3 à utiliser dans le traitement selon la revendication 2, dans lequel le traitement implique l'administration de l'anticorps pendant une période de 1 à 5 jours.

4. Anticorps anti-CD3 à utiliser dans le traitement selon l'une quelconque des revendications précédentes, pour le traitement de la synovite inflammatoire chronique.

5. Anticorps anti-CD3 à utiliser dans le traitement selon l'une quelconque des revendications précédentes, pour le traitement de la polyarthrite rhumatoïde.

6. Anticorps anti-CD3 à utiliser dans le traitement selon l'une quelconque des revendications précédentes, dans lequel le traitement donne lieu à une réduction de l'inflammation locale et de l'inflammation systémique.

7. Anticorps anti-CD3 à utiliser dans le traitement selon la revendication 6, dans lequel le traitement est **caractérisé par le fait que** l'on obtient une rétrocession substantielle de l'inflammation locale et de l'inflammation systémique.

8. Anticorps anti-CD3 à utiliser dans le traitement selon l'une quelconque des revendications précédentes, dans lequel le traitement implique une administration de l'anticorps à une dose totale de 50 à 100 mg.

9. Anticorps anti-CD3 à utiliser dans le traitement selon l'une quelconque des revendications précédentes, dans lequel le traitement implique l'administration de l'anticorps de manière conjointe avec un agent thérapeutique supplémentaire pour le traitement de l'inflammation articulaire chronique.

10. Anticorps anti-CD3 à utiliser dans le traitement selon la revendication 9, dans lequel le traitement implique l'administration de l'anticorps avec un stéroïde ou un anticorps anti-TNF.

11. Anticorps anti-CD3 à utiliser dans le traitement selon l'une quelconque des revendications 1 à 10, dans lequel l'anticorps se présente sous la forme d'une composition pharmaceutique comprenant également un support ou un diluant physiologiquement acceptable.


### SEQUENCE LISTING

<110> Isis Innovation Limited
<120> TREATMENT OF CHRONIC JOINT INFLAMMATION
<130> JIM/G17311WO
<160> 18
<170> PatentIn version 3.1
<210> 1
   <211> 5
   <212> PRT
   <213> rattus
<400> 1
   Ser Phe Pro Met Ala
   1               5
<210> 2
   <211> 17
   <212> PRT
   <213> rattus
<400> 2
   Thr Ile Ser Thr Ser Gly Gly Arg Thr Tyr Tyr Arg Asp Ser Val Lys
   1               5                   10                  15
   Gly
<210> 3
   <211> 10
   <212> PRT
   <213> rattus
<400> 3
   Phe Arg Gln Tyr Ser Gly Gly Phe Asp Tyr
   1 5 10
<210> 4
   <211> 13
   <212> PRT
   <213> rattus
<400> 4
   Thr Leu Ser Ser Gly Asn Ile Glu Asn Asn Tyr Val His
   1 5 10
<210> 5
   <211> 7
   <212> PRT
   <213> rattus
<400> 5
   Asp Asp Asp Lys Arg Pro Asp
   1 5
<210> 6
   <211> 9
   <212> PRT
   <213> rattus
<400> 6
   His Ser Tyr Val Ser Ser Phe Asn Val
   1 5
<210> 7
   <211> 30
   <212> PRT
   <213> rattus
<400> 7
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser
   20 25 30
<210> 8
   <211> 14
   <212> PRT
   <213> rattus
<400> 8
   Trp Val Arg Gln Ala Pro Gly Lys Gly Leu Glu Trp Val Ser
   1 5 10
<210> 9
   <211> 32
   <212> PRT
   <213> rattus
<400> 9
   Arg Phe Thr Ile Ser Arg Asp Asn Ser Lys Asn Thr Leu Tyr Leu Gln
   1 5 10 15
   Met Asn Ser Leu Arg Ala Glu Asp Thr Ala Val Tyr Tyr Cys Ala Lys
   20 25 30
<210> 10
   <211> 11
   <212> PRT
   <213> rattus
<400> 10
   Trp Gly Gln Gly Thr Leu Val Thr Val Ser Ser
   1 5 10
<210> 11
   <211> 119
   <212> PRT
   <213> rattus
<400> 11
<210> 12
   <211> 22
   <212> PRT
   <213> rattus
<400> 12
   Asp Phe Met Leu Thr Gln Pro His Ser Val Ser Glu Ser Pro Gly Lys
   1 5 10 15
   Thr Val Ile Ile Ser Cys
   20
<210> 13
   <211> 15
   <212> PRT
   <213> rattus
<400> 13
   Trp Tyr Gln Gln Arg Pro Gly Arg Ala Pro Thr Thr Val Ile Phe
   1 5 10 15
<210> 14
   <211> 34
   <212> PRT
   <213> rattus
<400> 14
   Gly Val Pro Asp Arg Phe Ser Gly Ser Ile Asp Arg Ser Ser Asn Ser
   1               5                   10                  15
   Ala Ser Leu Thr Ile Ser Gly Leu Gln Thr Glu Asp Glu Ala Asp Tyr
               20                  25                  30
   Tyr Cys
<210> 15
   <211> 10
   <212> PRT
   <213> rattus
<400> 15
   Phe Gly Gly Gly Thr Lys Leu Thr Val Leu
   1               5                   10
<210> 16
   <211> 110
   <212> PRT
   <213> rattus
<400> 16
   Asp Phe Met Leu Thr Gln Pro His Ser Val Ser Glu Ser Pro Gly Lys
   1 5 10 15
   Thr Val Ile Ile Ser Cys Thr Leu Ser Ser Gly Asn Ile Glu Asn Asn
   20 25 30
   Tyr Val His Trp Tyr Gln Gln Arg Pro Gly Arg Ala Pro Thr Thr Val
   35 40 45
   Ile Phe Asp Asp Asp Lys Arg Pro Asp Gly Val Pro Asp Arg Phe Ser
   50 55 60
   Gly Ser Ile Asp Arg Ser Ser Asn Ser Ala Ser Leu Thr Ile Ser Gly
   65 70 75 80
   Leu Gln Thr Glu Asp Glu Ala Asp Tyr Tyr Cys His Ser Tyr Val Ser
   85 90 95
   Ser Phe Asn Val Phe Gly Gly Gly Thr Lys Leu Thr Val Leu
   100 105 110
<210> 17
   <211> 36
   <212> DNA
   <213> rattus
<400> 17
   gactacaagc ttacacagga cctcaccatg cgatgg
   36
<210> 18
   <211> 36
<212> DNA
   <213> rattus
<400> 18
   gatgctgaat tctgcagctc tagtctcccg tggtgg
   36